# EUROPÄISCHE PATENTSCHRIFT

(11) **EP 1 519 708 B1**
(45) Veröffentlichungstag und Bekanntmachung des Hinweises auf die Patenterteilung: **02.04.2008**
(21) Anmeldenummer: 03762421.0
(22) Anmeldetag: 23.06.2003
(51) Int. Cl.: A61K 8/14, A61K 8/97, A61K 8/99, A61Q 19/08

(54) **ANTI-HAUTALTERUNGSKOSMETIKUM**
ANTI-AGEING SKIN COSMETIC
PRODUIT COSMETIQUE ANTI-VIEILLISSEMENT CUTANE

(30) Priorität: 08.07.2002 DE 10231468
(43) Veröffentlichungstag der Anmeldung: 06.04.2005
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2003/002157
(87) Internationale Veröffentlichungsnummer: WO 2004/004673

(56) Entgegenhaltungen:
- WO-A-00/41674
- WO-A-02/49593
- WO-A-98/26755
- WO-A-99/66881
- CELLEX-C PRODUCTS, [Online] XP002261941 Gefunden im Internet: <URL:http://natural-skin-care-company.com/ cellex-c-speed-peel.html> [gefunden am 2003-11-18]

## Beschreibung

Die Erfindung betrifft ein Kosmetikum, das auf Basis verschiedener natürlicher Rohstoffe, gegen Alterungsprozesse bei menschlicher Haut eingesetzt werden kann.

Aus dem Stand der Technik sind bereits eine große Anzahl von Kosmetika bekannt, die in irgendeiner Weise pflanzliche Rohstoffe in Form von ölen oder Extrakten enthalten. Dabei werden meist die vorteilhaften bekannten Wirkungen einzelner Pflanzen genutzt, um eine entsprechende Gesamtwirkung zu erzielen. So wird z.B. die Wirkung von Rosmarin als öl oder als Extrakt der Blätter auch in Kombination mit anderen Pflanzenextrakten häufiger genutzt, um die krampflösenden und kreislaufanregenden Eigenschaften einzubeziehen. Auch Algenextrakte werden in maritimen Kosmetika häufiger eingesetzt. Beispielsweise beschreibt die WO98/2675 ein Präparat, das Extrakte der Alge Laminaria saccharina, Wurzelextrakte von Lilium candididum und Extrakte von Glycyrrhyza glabra enthält und dabei eine besondere Hautreinigungswirkung erzielt. Rosamrinextrakt wird in der WO 00/41674 als Mittel gegen die Hautalterung eingesetzt.

Der Erfindung liegt die Aufgabe zugrunde, ein verbessertes Kosmetikum in Bezug auf die Hautalterung bereitzustellen.
Eine weitere Aufgabe besteht darin, ein zugleich in der Feuchthaltewirkung verbessertes Kosmetikum zu entwickeln.

Erfindungsgemäß bereitgestellt wird daher ein Anti-Hautalterungskosmetikum, das enthält
0,1 bis 5 Gew-% eines Extraktes aus einem Gemisch von Feigenblättern und -früchten;
0,1 bis 3 Gew-% eines Extraktes aus Granatapfelfrüchten;
0,001 bis 0,5 Gew-% eines gemahlenen Trockengemisches von Stengeln und Blättern von Rosmarin;
0,01 bis 3 Gew-% Liposomen, enthaltend einen Extrakt von geschälten Zuckermelonen;
0,1 bis 5 Gew-% Liposomen, enthaltend einen Planktonextrakt mit einem Gehalt des Enzyms Photolyase;
0,1 bis 5 Gew-% Liposomen, enthaltend 0,1 bis 0,5 Gew-%, bezogen auf das Liposomengewicht, eines Micrococcus-Lysats mit einem Gehalt des Enzyms UV-Endonuclease; und
bis 100 Gew-% weitere Wirkstoffe, Trägerstöffe, Hilfsstoffe oder Gemische davon,
wobei alle Prozentangaben auf das Gesamtgewicht des Kosmetikums bezogen sind.

Der eingesetzte Feigenextrakt ist ein Extrakt, der aus einem Gemisch von Blättern und Früchten der Feige Ficus carica besteht, wobei der Anteil beider Bestandteile im Verhältnis 25-75 : 75-25 liegen kann. Es handelt sich um einen Kaltextrakt (Extraktionstemperatur 10-30 °C) mit einem Wasser/Propylenglycol-Extraktionsmittel. Der Extrakt enthält wirksame Anteile des proteolytischen Enzyms Ficin und trägt zur Zellerneuerung bei.

Der Granatapfelextrakt von Punica granatum ist ein Extrakt auf Basis Wasser und Propylenglycol und regt die Mikrozirkulation der Haut an.

Rosmarin Rosmarinus officinalis wird anders als bekannt nicht in Form eines Extraktes oder öles sondern als gemahlenes Trockengemisch von Stengeln und Blättern eingesetzt. Ein solches Gemisch hat überraschenderweise eine hohe Aktivität zur Bindung freier Radikale und damit einen sehr hohen Radikalschutzfaktor (RPF).

Der Extrakt von Zuckermelonen Cucumis melo, der mit Wasser bei 10 bis 30 °C aus der Melone ohne deren äußerster Schale gewonnen wird, trägt zur Verringerung des transepidermalen Wasserverlustes (TEWL) bei und wird vorteilhaft über solche Liposome in das Kosmetikum eingebracht, deren äußere Hülle aus Phospholipiden und Olivenöl bestehen. Durch diese besondere Liposomeform wird die Textur des Kosmetikums deutlich verbessert.

Der über Liposome eingebrachte Planktonextrakt wird z.B. aus Cyanobakterien der Gattung Anacystes nidulans gewonnen und enthält eine wirksame Menge des Enzyms Photolyase. Dieses Enzym trägt dazu bei, daß infolge von UV-Einstrahlung entstandene Cyclobutanpyrimidin-Dimere unter Einbeziehung von Tageslicht wieder aufgespalten werden und somit ein Reparatureffekt für die Haut erreicht wird.

Besonders vorteilhaft ist die Verkapselung des Planktonextraktes in speziellen Liposomen, die aus drei Arten von Phospholipiden gebildet werden: aus Phosphatidylethanolamin, Phosphatidylcholin/ölsäure und Cholesterylhemisuccinat. Derartige Liposomen zeigen eine hohe Penetrationskraft in Keratinocyten und setzen ihren Inhalt in Abhängigkeit von einer pH-Absenkung von z.B. pH 6 auf pH 4 schlagartig frei. Ein bevorzugtes Produkt, das den Planktonextrakt enthält, ist Photosomes^{®} von Barnet Products Corp., NJ/USA.

Ebenfalls über Liposome wird ein Micrococcus-Lysat mit dem Enzym UV-Endonuclease eingebracht, das langanhaltende, hautreparierende Wirkungen hat. Die Liposome können die für den Planktonextrakt beschriebene vorteilhafte Ausführungsform haben. Ein bevorzugtes Produkt ist Ultrasomes^{®} von Barnet Products Corp., NJ/USA.

Die von dem erfindungsgemäßen Anti-Hautalterungskosmetikum auf Grund einiger Bestandteile zu erwartende Wirkung gegen Hautalterung wird durch eine synergistische Gesamtwirkung deutlich übertroffen. Darüber hinaus zeigt das Kosmetikum eine ausgezeichnete Feuchtigkeitsanreicherung in der Haut, die ebenfalls von den Grundbestandteilen allein nicht zu erwarten war.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdikkungsmittel, Duftstoffe, Alkohole, Polyole, Elektrolyte, Gelbildner, öle, Polymere, Copolymere, Emulgatoren, Wachse, Stabilisatoren.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit. Bevorzugt ist Carbomer und Hydroxyethylcellulose.

Die Wachse können ausgewählt werden unter natürlichen pflanzlichen Wachsen, tierischen Wachsen, natürlichen und synthetische Mineralwachsen und synthetischen Wachsen. Dazu gehören beispielsweise Carnaubawachs, Candellilawachs, Ozokerit, Bienenwachs, Montanwachs, Wollwachs, Ceresin, Mikrowachse, Hartparaffin, Petrolatum, Siliconwachs, Polyethylenglycol- oder - glycolesterwachse. Bevorzugt sind niedrigviskose Siliconwachse mit Viskositäten bis etwa 1000 Pa·s.

Als Feuchthaltemittel können eingesetzt werden Glycerin, Butylenglycol, Propylenglycol und Gemische davon. Bevorzugt ist Glycerin.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt ist 2-Hydroxy-4-methoxybenzophenon-5-sulfonsäure (Benzophenone-4) als wasser- und alkohollöslicher Breitbandfilter mit Gehalten von 0,1 bis 0,5 Gew-%.

Das erfindungsgemäße Kosmetikum kann auch ein- und/oder mehrwertige Alkohole enthalten, wie Ethanol, Propylenglycol, Dipropylenglycol, Ethylenglycol, Isoprenglycol, Butylenglycole, Sorbitol und Gemische davon. Der Anteil des Alkanols und/oder Polyols liegt vorteilhaft im Bereich von 5 bis 20 Gew-%.

Die für die Erfindung eingesetzten öle können übliche kosmetische öle mit niedrigen Viskositäten sein wie Jojobaöl und Siliconöle bis etwa 1000 Pa·s.

Das Kosmetikum kann Farbstoffe oder Pigmente, Pigmentgemische oder Pulver mit pigmentartiger Wirkung enthalten. Pigmente können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Als weitere Wirkstoffe können Antioxidationsmittel und Radikalfänger enthalten. Zu derartigen Substanzen gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate.

Ein besonders bevorzugter Wirkstoff ist eine Wirkstoffzubereitung, enthaltend (a) einen durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, wobei der Gehalt von (a), das in einer an Mikrokapseln gebundenen Wirkstoffkonzentration von 2 Gew-% vorliegt, im Bereich von 0,1 bis 10 Gew- % liegt; (b) ein durch Extraktion gewonnener Seidenraupenextrakt, der das Peptid Cecropine, Aminosäuren und ein Vitamingemisch enthält, wobei der Gehalt von (b) im Bereich von 0,1 bis 10 Gew- % liegt; (c) ein nichtionisches, kationisches oder anionisches Hydro-Gel oder Gemisch von Hydro-Gelen mit einem Gehalt von (c) im Bereich von 0,1 bis 5 Gew-%; wobei die Wirkstoffe (a) und (b) zusammen mit dem Gel (c) und einem oder mehreren Phospholipiden mit einem Anteil von 0,1 bis 30 Gew-% und mit Wasser einen Assoziationskomplex bilden und die Prozentangaben jeweils bezogen auf das Gesamtgewicht der Wirkstoffzubereitung sind.

In einer bevorzugten Ausführungsform besteht das Anti-Hautalterungskosmetikum aus 0,1 bis 1 Gew-% eines Extraktes aus Feigenblättern und -früchten;
0,1 bis 1 Gew-% eines Extraktes aus Granatapfelfrüchten;
0,001 bis 0,1 Gew-% eines gemahlenen Trockengemisches von Stengeln und Blättern von Rosmarin;
0,01 bis 1 Gew-% eines Extraktes von geschälten Zuckermelonen; 0,1 bis 1 Gew-% Liposomen, enthaltend einen Planktonextrakt mit einem Gehalt des Enzyms Photolyase;
0,1 bis 1 Gew-% Liposomen, enthaltend 0,1 bis 0,5 Gew-%, bezogen auf das Liposomengewicht, eines Micrococcus-Lysats mit einem Gehalt des Enzyms UV-Endonuclease;
60 bis 80 Gew-% Wasser;
0,1 bis 1 Gew-% eines siliconwachses;
0,3-0,7 Gew-% eines Konservierungsmittels oder Konservierungsmittelgemisches;
0,01 bis 0,5 Gew-% Na-EDTA (Tetranatrium-ethylendiamintetraessigsäure);
1 bis 10 Gew-% Butane Diol 1.3/1,3 Butylene Glycol U-Pur:
0,1 bis 0,5 Gew-% Carbomer;
0,1 bis 0,5 Gew-% Hydroxyethyl Cellulose;
0,1 bis 0,5 Triethanolamine;
0,1 bis 1 Gew-% Xanthan Gum;
1 bis 5 Gew-% Glycerine;
1 bis 15 Gew-% Ethanol;
0,1 bis 0,5 Gew-% einer Wirkstoffzubereitung, enthaltend
(a) ein durch Extraktion der Rinde von Quebracho blanco und nachfolgender enzymatischer Hydrolyse gewonnenes Produkt, das wenigstens 90 Gew- % Proanthocyanidin-Oligomere und höchstens 10 Gew-% Gallussäure enthält, wobei der Gehalt von (a), das in einer an Mikrokapseln gebundenen Wirkstoffkonzentration von 2 Gew-% vorliegt, im Bereich von 0,1 bis 10 Gew- % liegt; (b) ein durch Extraktion gewonnener Seidenraupenextrakt, der das Peptid cecropine, Aminosäuren und ein Vitamingemisch enthält, wobei der Gehalt von (b) im Bereich von 0,1 bis 10 Gew- % liegt; (c) ein nichtionisches, kationisches oder anionisches Hydro-Gel oder Gemisch von Hydro-Gelen mit einem Gehalt von (c) im Bereich von 0,1 bis 5 Gew-%; wobei die Wirkstoffe (a) und (b) zusammen mit dem Gel (c) und einem oder mehreren Phospholipiden mit einem Anteil von 0,1 bis 30 Gew-% und mit Wasser einen Assoziationskomplex bilden und die Prozentangaben jeweils bezogen auf das Gesamtgewicht der Wirkstoffzubereitung sind;
0,2 bis 0,5 Gew-% PEG 40;
0,1-0,5 Gew-% Benzophenone-4; und gegebenenfalls jeweils 0,1
bis 1 Gew-% Farbe und Parfüm,
wobei alle Prozentangaben, sofern nicht anders angegeben, auf das Gesamtgewicht des kosmetischen Komplexes bezogen sind.

Die bevorzugte Ausführungsform zeigt insgesamt eine sehr gute Anti-Alterungswirkung, was durch Vergleichsversuche nachgewiesen werden konnte. Sie hat auch eine ausgezeichnete Feuchthaltewirkung gemäß durchgeführten Corneometetmessungen.

Das erfindungsgemäße Kosmetikum hat einen Radikalschutzfaktor im Bereich von 40 bis 100 x 10¹⁴ Radikale/mg. Der Radikalschutzfaktor (RPF) bestimmt die Aktivität zur Bindung freier Radikale durch ein Antioxidationsmittel gegenüber einer Testsubstanz. Die Messung erfolgte wie in WO 99/66881 beschrieben.

Die Erfindung soll nachstehend durch Beispiele näher erläutert werden. Alle Angaben erfolgen in Gewichtsprozent, sofern nichts anderes angegeben ist.

**Beispiel 1 Gesichtsserum I**

| | |
|---|---|
| Wasser | q.s. ad 100 |
| Siliconwachs | 0,5 |
| Konservierungsmittel | 0,5 |
| Tetranatrium-EDTA | 0,1 |
| Butane Diol 1.3/Butylene Glycol U-Pur | 5,0 |
| Carbomer | 0,2 |
| Hydroxyethylcellulose | 0,2 |
| Triethanolamin | 0,2 |
| Xanthan Gum | 0,6 |
| Glycerin | 1,5 |
| Ethanol | 10,0 |
| Feigenextrakt | 0,5 |
| Granatapfelextrakt | 0,5 |
| Wirkstoffzubereitung* | 0,1 |
| Rosmarinpulver | 0,001 |
| Melonen-Liposome | 0,1 |
| Parfüm | 0,1 |
| PEG 40 | 0,3 |
| Farbstoff | q.s. |
| Plankton-Liposome (Photosomes) | 0,1 |
| Lysat-Liposome (Ultrasomes) | 0,1 |
| Benzophenone-4 | 0,1 |

| | |
|---|---|
| * mit Quebracho blanco (2 %), Seidenraupenextrakt (1 %), trockenes Gel (1 %), Phospholipiden (7 %) und Wasser (89 %) ; Herstellung wie in WO99/66881 Beispiel 1 beschrieben. | |

Die Herstellung des Serums erfolgte unter 45 °C. In das erwärmte Wasser wurde Wachs gegeben und verrührt. Danach wurde Carbomer zugegeben, gut verrührt und neutralisiert. Im Anschluß daran erfolgte bei Raumtemperatur die Zugabe der restlichen Rohstoffe unter Rühren. Schließlich wurde das Gemisch homogenisiert. Das Serum hatte einen RPF von 67

**Beispiel 2 Gesichtsserum II**

| | |
|---|---|
| Wasser | q.s. ad 100 |
| Siliconwachs | 0,9 |
| Konservierungsmittel | 0,3 |
| Tetranatrium-EDTA | 0,2 |
| Butane Diol 1.3/Butylene Glycol U-Pur | 7,0 |
| Carbomer | 0,3 |
| Hydroxyethylcellulose | 0,1 |
| Triethanolamin | 0,3 |
| Xanthan Gum | 0,5 |
| Glycerin | 1,0 |
| Ethanol | 5,0 |
| Feigenextrakt | 1,0 |
| Granatapfelextrakt | 1,5 |
| Wirkstoffzubereitung* | 0,5 |
| Rosmarinpulver | 0,01 |
| Melonen-Liposome | 2,0 |
| Parfüm | 0,01 |
| PEG 40 | 0,1 |
| Farbstoff | q.s. |
| Plankton-Liposome (Photosomes) | 0,5 |
| Lysat-Liposome (Ultrasomes) | 0,5 |
| Benzophenone-4 | 0,2 |
| Silicone | 2,0 |

| | |
|---|---|
| * mit Quebracho blanco (2 %), Seidenraupenextrakt (1 %), trockenes Gel (1 %), Phospholipiden (7 %) und Wasser (89 %) ; Herstellung wie in WO99/66881 Beispiel 1 beschrieben. | |

Die Herstellung der Lotion erfolgte wie im Beispiel 1. Das Serum hatte einen RPF von 69.

### Beispiel 3 (Testbeispiel)

### (A) Anti-Alterungswirkung

Es wurden Consumer-Tests bei 150 Verbrauchern durch 4-wöchige Anwendung des Serums nach Beispiel 1 im Gesicht durchgeführt und eine Auswertung durch Fragebogen.

Von den Befragten, die das Produkt regelmäßig morgens und abends angewendet hatten (82%), bewerteten 39 % mit "fühlbares Ergebnis erzielt", 32 % mit "teilweise fühlbares Ergebnis erzielt" und 11 % "kein fühlbares Ergebnis erzielt".

Im einzelnen wurden folgende Ergebinise erzielt:

| | |
|---|---|
| Verbesserung der Hautelastizität | nach 1 Woche bei 77%* |
| | nach 2 Wochen bei 87% |
| | nach 3 Wochen bei 84% |
| Feinlinienreduzierung | nach 1 Woche bei 76%* |
| | nach 2 Wochen bei 81% |
| | nach 3 Wochen bei 83% |
| Aussehen der Haut (Firmness) | nach 1 Woche bei 81%* |
| | nach 2 Wochen bei 84% |
| | nach 3 Wochen bei 84% |

| | |
|---|---|
| * der Probanden | |

Besonders überraschen war die Tatsache, daß 96 % der Probanden einen sofortigen Effekt schon nach 1-2 Tagen feststellen konnten und die Wirkung nach 1 Woche bei einem hohen Prozentsatz deutlich erkennbar war.

Ein Vergleichsversuch mit 20 Probanden, die das Serum von Beispiel 1 in gleicher Weise benutzten, wobei jedoch im Serum nicht enthalten waren Rosmarinpulver und Granatapfelextrakt, ergab eine Verlzögerung des Verbesserungseffektes und niedrigere Werte:

| | |
|---|---|
| Verbesserung der Hautelastizität | nach 1 Woche bei 52%* |
| | nach 2 Wochen bei 58% |
| | nach 3 Wochen bei 51% |
| Feinlinienreduzierung | nach 1 Woche bei 33%* |
| | nach 2 Wochen bei 43% |
| | nach 3 Wochen bei 45% |
| Aussehen der Haut (Firmness) | nach 1 Woche bei 45%* |
| | nach 2 Wochen bei 53% |
| | nach 3 Wochen bei 58% |

| | |
|---|---|
| * der Probanden | |

Diese deutlichen Unterschiede lassen auf einen Synergismus schließen.

### (B) Feuchthaltewirkung

Im Zusammenhang mit dem Consumertest gemäß (A) wurde die Feuchthaltewirkung bei den Verbrauchern bewertet mit den Stufen:
- wenig feuchthaltend
- normal feuchthaltend
- langandauernd feuchthaltend (≥ 24 Stunden).

Von 100 % der Verbraucher bewerteten 18 % die Wirkung als "normal feuchthaltend" und 79 % als "langandauernd feuchthaltend". Von 3 % gab es keine Aussage.

Dieses Ergebnisse belegen eine ausgezeichnete Feuchthaltewirkung zusammen mit einer ebenfalls sehr guten Anti-Alterungswirkung.

## Patentansprüche

1. Anti-Hautalterungskosmetikum, **dadurch gekennzeichnet, daß** es enthält
0,1 bis 5 Gew-% eines Extraktes aus einem Gemisch von Feigenblättern und -früchten;
0,1 bis 3 Gew-% eines Extraktes aus Granatapfelfrüchten;
0,001 bis 0,5 Gew-% eines gemahlenen Trockengemisches von Stengeln und Blättern von Rosmarin;
0,01 bis 3 Gew-% Liposomen, enthaltend einen Extrakt von geschälten Zuckermelonen;
0,1 bis 5 Gew-% Liposomen, enthaltend einen Planktonextrakt mit einem Gehalt des Enzyms Photolyase;
0,1 bis 5 Gew-% Liposomen, enthaltend 0,1 bis 0,5 Gew-%, bezogen auf das Liposomengewicht, eines Micrococcus-Lysats mit einem Gehalt des Enzyms UV-Endonuclease; und
bis 100 Gew-% weitere Wirkstoffe, Trägerstoffe, Hilfsstoffe oder Gemische davon,
wobei alle Prozentangaben auf das Gesamtgewicht des Kosmetikums bezogen sind.

2. Kosmetikum nach Anspruch 1, **dadurch gekennzeichnet, daß** die Liposomen, die den Zuckermelonenextrakt enthalten, aus Phospholipiden und Olivenöl aufgebaut sind.

3. Kosmetikum nach Anspruch 1, **dadurch gekennzeichnet, daß** der Planktonextrakt aus dem Cyanobakterium Anacystes nidulans herrührt.

4. Kosmetikum nach Anspruch 1, **dadurch gekennzeichnet, daß** die Liposomen, die den Planktonextrakt enthalten, aus einem Gemisch von Phosphatidyl-ethanolamin, Phosphatidylcholin, ölsäure und Cholesteryl-hemisuccinat bestehen.

5. Anti-Hautalterungskosmetikum nach Anspruch 1, **dadurch gekennzeichnet, daß** es enthält
0,1 bis 1 Gew-% eines Extraktes aus Feigenblättern und -früchten;
0,1 bis 1 Gew-% eines Extraktes aus Granatapfelfrüchten;
0,001 bis 0,1 Gew-% eines gemahlenen Trockengemisches von Stengeln und Blättern von Rosmarin;
0,01 bis 1 Gew-% eines Extraktes von geschälten Zuckermelonen;
0,1 bis 1 Gew-% Liposomen, enthaltend einen Planktonextrakt mit einem Gehalt des Enzyms Photolyase;
0,1 bis 1 Gew-% Liposomen, enthaltend 0,1 bis 0,5 Gew-%, bezogen auf das Liposomengewicht, eines Micrococcus-Lysats mit einem Gehalt des Enzyms UV-Endonuclease;
60 bis 80 Gew-% Wasser;
0,1 bis 1 Gew-% eines Siliconwachses;
0,3-0,7 Gew-% eines Konservierungsmittels oder Konservierungsmittelgemisches;
0,01 bis 0,5 Gew-% Na-EDTA (Tetranatrium-ethylendiamintetraessigsäure);
1 bis 10 Gew-% Butane Diol 1.3/1,3 Butylene Glycol U-Pur:
0,1 bis 0,5 Gew-% Carbomer;
0,1 bis 0,5 Gew-% Hydroxyethyl Cellulose;
0,1 bis 0,5 Triethanolamine;
0,1 bis 1 Gew-% Xanthan Gum;
1 bis 5 Gew-% Glycerine;
1 bis 15 Gew-% Ethanol;
0,1 bis 0,5 Gew-% einer Wirkstoffzubereitung, enthaltend
(a) ein durch Extraktion der Rinde von Quebracho blanco und nachfolgender enzymatischer Hydrolyse gewonnenes Produkt, das wenigstens 90 Gew- % Proanthocyanidin-Oligomere und höchstens 10 Gew-% Gallussäure enthält, wobei der Gehalt von (a), das in einer an Mikrokapseln gebundenen Wirkstoffkonzentration von 2 Gew-% vorliegt; (b) ein durch Extraktion gewonnener Seidenraupenextrakt, der das Peptid Cecropine, Aminosäuren und ein Vitamingemisch enthält, wobei der Gehalt von (b) im Bereich von 0,1 bis 10 Gew- % liegt;
(c) ein nichtionisches, kationisches oder anionisches Hydro-Gel oder Gemisch von Hydro-Gelen mit einem Gehalt von (c) im Bereich von 0,1 bis 5 Gew-%; wobei die Wirkstoffe (a) und (b) zusammen mit dem Gel (c) und einem oder mehreren Phospholipiden mit einem Anteil von 0,1 bis 30 Gew-% und mit Wasser einen Assoziationskomplex bilden und die Prozentangaben jeweils bezogen auf das Gesamtgewicht der Wirkstoffzubereitung sind;
0,2 bis 0,5 Gew-% PEG 40;
0,1-0,5 Gew-% Benzophenone-4; und gegebenenfalls jeweils 0,1 bis 1 Gew-% Farbe und Parfüm,
wobei alle Prozentangaben, sofern nicht anders angegeben, auf das Gesamtgewicht des kosmetischen Komplexes bezogen sind.

6. Verwendung eines Kosmetikums nach einem der Ansprüche 1 bis 5 zur Faltenreduzierung und zur Verlängerung des Zeitraumes für eine sichtbar faltenfreie Haut.

## Claims

1. An anti-ageing skin cosmetic comprising
0.1 to 5% by weight of an extract from a mixture of fig leaves and fruits;
0.1 to 3% by weight of an extract from pomegranate fruits;
0.001 to 0.5% by weight of a ground dry mixture of rosemary stems and leaves;
0.01 to 3% by weight of liposomes containing an extract from peeled musk melons;
0.1 to 5% by weight of liposomes containing a plankton extract containing the photolyase enzyme;
0.1 to 5% by weight of liposomes containing 0.1 to 0.5% by weight, in relation to the liposome weight, of a micrococcus lysate containing the UV-endonuclease enzyme; and
up to 100% by weight, other active substances, carrier substances, adjuvants or mixtures thereof,
all percentages being relative to the cosmetic's total weight.

2. A cosmetic according to claim 1, wherein the liposomes containing the musk melon extract are made up of phospholipids and olive oil.

3. A cosmetic according to claim 1, wherein the plankton extract stems from the Anacystes nidulans cyanobacterium.

4. A cosmetic according to claim 1, wherein the liposomes containing the plankton extract consist of a mixture of phosphatidyl ethanolamine, phosphatidylcholine, oleic acid and cholesteryl hemisuccinate.

5. An anti-ageing skin cosmetic according to claim 1, wherein said cosmetic contains
0.1 to 1% by weight of an extract from fig leaves and fruits;
0.1 to 1% by weight of an extract from pomegranate fruits;
0.001 to 0.1% by weight of a ground dry mixture of rosemary stems and leaves;
0.01 to 1% by weight of an extract from peeled musk melons;
0.1 to 1% by weight of liposomes containing a plankton extract containing the photolyase enzyme;
0.1 to 1% by weight of liposomes containing 0.1 to 0.5% by weight, in relation to the liposome weight, of a micrococcus lysate containing the UV-endonuclease enzyme;
60 to 80% by weight water;
0.1 to 1% by weight of a silicone wax;
0.3 to 0.7% by weight of a preservative or preservative mixture;
0.01 to 0.5% by weight Na-EDTA (Tetrasodium Ethylenediamine Tetraacetic Acid);
1 to 10% by weight Butane Diol 1.3/1,3 Butylene Glycol U-Pur;
0.1 to 0.5% by weight Carbomer;
0.1 to 0.5% by weight Hydroxyethyl Cellulose;
0.1 to 0.5% by weight Triethanolamine;
0.1 to 1% by weight Xanthan Gum;
1 to 5% by weight Glycerine;
1 to 15% by weight Ethanol;
0.1 to 0.5% by weight of an active formulation containing
(a) a product obtained by extracting the bark of Quebracho blanco and subsequent enzymatic hydrolysis, which product contains at least 90% by weight proanthocyanidine oligomers and max. 10% by weight gallic acid, (a), whose active substance concentration is 2% by weight, bound to microcapsules, being contained in an amount ranging between 0.1 and 10% by weight;
(b) a silkworm extract obtained by extraction, which extract contains the cecropine peptide, amino acids and a vitamin mixture, (b) being contained in an amount ranging between 0.1 and 10% by weight;
(c) a non-ionic, cationic or anionic hydro-gel or hydrogel mixture, (c) being contained in an amount ranging between 0.1 and 5% by weight; wherein the active substances (a) and (b) form an association complex together with the gel (c) and one or several phospholipid(s) contained in an amount between 0.1 and 30% by weight and with water, and each of the aforesaid percentages is in relation to the total weight of the active formulation;
0.2 to 0.5% by weight PEG 40;
0.1 to 0.5% by weight Benzophenone-4; and optionally 0.1 to 1% by weight of colourant and perfume respectively,
all percentages being relative to the total weight of the cosmetic complex unless indicated otherwise.

6. The use of a cosmetic according to any one of claims 1 to 5 for reducing wrinkles and for prolonging the period for a visibly wrinkle-free skin.

## Revendications

1. Agent cosmétique anti-vieillissement cutané, **caractérisé en ce que**
qu'il comprend
de 0,1 à 5 % en poids d'un extrait issu d'un mélange de feuilles et de fruits de figues ;
de 0,1 à 3 % en poids d'un extrait issu de fruits de grenade ;
de 0,001 à 0,5 % en poids d'un mélange moulu sec de tiges et de feuilles du romarin ;
de 0,01 à 3 % en poids de liposomes, contenant un extrait de pastèques pelées ;
de 0,1 à 5 % en poids de liposomes, comprenant un extrait de plancton ayant une teneur de l'enzyme photolyase ;
de 0,1 à 5 % en poids de liposomes, comprenant de 0,1 à 0,5 % en poids, par rapport au poids des liposomes, d'un lysat de micrococcus ayant une teneur de l'enzyme UV-endonucléase ; et
jusqu'à 100 % en poids d'autres agents actifs, d'excipients, d'adjuvants ou de mélanges de ces derniers,
toutes les indications en pour cent se rapportant au poids total de l'agent cosmétique.

2. Agent cosmétique selon la revendication 1, **caractérisé en ce que** les liposomes, qui contiennent l'extrait de pastèques, sont constitués de phospholipides et d'huile d'olive.

3. Agent cosmétique selon la revendication 1, **caractérisé en ce que** l'extrait de plancton provient de Cyanobakterium Anacystes nidulans.

4. Agent cosmétique selon la revendication 1, **caractérisé en ce que** les liposomes, qui contiennent l'extrait de plancton, se composent d'un mélange de phosphatidyl-éthanolamine, de phosphatidyl-choline, d'acide oléique et d'hémisuccinate de cholestéryle.

5. Agent anti-vieillissement cutané selon la revendication 1, **caractérisé en ce qu'**il comprend
de 0,1 à 1 % en poids d'un extrait issu de fruits de figues ;
de 0,1 à 1 % en poids d'un extrait issu de fruits de grenade ;
de 0,001 à 0,1 % en poids d'un mélange moulu sec de tiges et de feuilles du romarin ;
de 0,01 à 1% d'un extrait de pastèques pelées ;
de 0,1 à 1 % en poids des liposomes, comprenant un extrait de plancton ayant une teneur de l'enzyme photolyase ;
de 0,1 à 1 % en poids des liposomes, comprenant de 0,1 à 0,5 % en poids, par rapport au poids des liposomes, d'un lysat de micrococcus ayant une teneur de l'enzyme UV-endonucléase ; et
de 60 à 80 % en poids d'eau ;
de 0,1 à 1% en poids d'une cire de silicones,
0,3-0,7 % en poids d'un agent de conservation ou d'un mélange d'agents de conservation ;
de 0,01 à 0,5 % en poids de Na-EDTA (acide éthylène-diaminetétra-acétique tétrasodique) ;
de 1 à 10 % en poids de butanediol-1,3/de 1,3-butylèneglycol U-Pur ;
de 0,1 à 0,5 % en poids de carbomère ;
de 0,1 à 0,5 % en poids d'hydroxyéthylcellulose ;
de 0,1 à 0,5 de triéthanolamine ;
de 0,1 à 1 % en poids de gomme de xanthane ;
de 1 à 5 % en poids de glycérine;
de 1 à 15 % en poids d'éthanol ;
de 0,1 à 0,5 % en poids d'une préparation d'agents actifs, contenant
(a) un produit obtenu par extraction de l'écorce de Quebracho blanco et par hydrolyse enzymatique subséquente, qui contient au moins 90 % en poids d'oligomères de proanthocyanidine et d'au plus 10 % en poids d'acide gallique, la teneur en (a), étant présente dans une concentration d'agents actifs, liés dans des microcapsules, de 2 % en poids ;
(b) un extrait de vers à soie obtenu par extraction, qui contient la peptide cécropine, des aminoacides et un mélange de vitamines, la teneur en (b) se situant dans le domaine de 0,1 à 10 % en poids ;
(c) un hydrogel non ionique, cationique ou anionique ou un mélange d'hydrogels ayant une teneur en (c) dans le domaine de 0,1 à 5 % en poids ; les agents actifs (a) et (b), conjointement au gel (c) et à un ou plusieurs phospholipides ayant une proportion de 0,1 à 30 % en poids et à de l'eau, formant un complexe d'association et les indications en pour cent se rapportant à chaque fois au poids total de la préparation d'agents actifs ;
de 0,2 à 80 % en poids de PEG 40 ;
de 0,1-0,5 % en poids de benzophénone-4 ; et, le cas échéant, à chaque fois, de 0,1 à 1 % en poids de colorant et de parfum,
toutes les indications en pour cent, sauf indication expresse du contraire, se rapportant au poids total du complexe cosmétique.

6. Utilisation d'un agent cosmétique selon l'une quelconque des revendications 1 à 5 en vue de la réduction des rides et de la prolongation de l'intervalle de temps pour une peau visiblement exempte de rides.
